# EUROPEAN PATENT APPLICATION

(11) **EP 3 034 638 A1**
(43) Date of publication of application: **22.06.2016**
(21) Application number: 14835886.4
(22) Date of filing: 12.08.2014
(51) Int. Cl.: C22C 19/03, A61F 2/82, A61L 29/00, A61L 31/00, A61M 25/00, A61M 25/14, C22F 1/10, C22F 1/00

(54) **MEDICAL Ti-Ni ALLOY**

(30) Priority: 12.08.2013 JP 2013177285
(71) Applicant: Tohoku University, Sendai-shi, Miyagi 980-8577 (JP)
(72) Inventor: Yamauchi Kiyoshi, Sendai-shi Miyagi 980-8577 (JP); Suzuki Masao, Sendai-shi Miyagi 980-8579 (JP); Uegaki Yukihiro, Sendai-shi Miyagi 980-8579 (JP)
(74) Representative: Hoffmann Eitle
(86) International application number: PCT/JP2014/071338
(87) International publication number: WO 2015/022969

(57) **Abstract**

[Problem to be solved] The present invention provides a medical Ti-Ni alloy largely including a TiNi single phase, in which both strength and plasticity of the metal material at the body temperature are maintained, and formation of a knife-edged fracture surface when bending and breaking is less significant. The medical Ti-Ni alloy can be used for cores of catheters, guide wires and the like.

[Solution] The present inventors find that above objective can be achieved by using an alloy largely including a TiNi single phase. Then the present invention has been completed.

## Description

### Field

The present invention relates to a medical alloy which can be used in a human body, and a medical catheterization device including the medical alloy.

### Background

An endovascular treatment, which is also referred to as a catheter treatment, involves inserting a tube called a catheter into an affected area through a blood vessel at a groin or wrist to perform percutaneous transluminal angioplasty. In recent years, this medical technology is rapidly advancing as less-invasive medical treatments become popular in view of reducing patient burden.

Main devices for the catheter treatment are a guide wire, a catheter and a stent.

A guide wire is first inserted into the body, and serves as a guide for introducing a catheter into an affected area. The main required functions are the softness of a front end part for not causing damage to a blood vessel, the pushability of a base part to allow passage through a curved blood vessel and the shape restorability after passing a branched blood vessel. A core may be a stainless steel wire subjected to severe plastic deformation or a superelastic Ti-Ni alloy. Stainless steel is better in terms of stiffness/pushability while a Ti-Ni alloy is better in terms of softness/restorability. In recent years, a guide wire with a hybrid core including a base part of stainless steel and a front end part of a Ti-Ni alloy has been used for medical use.

A catheter is a tube for delivering a medical liquid and device required to diagnose/treat an affected area. Pressure resistance to flush of a medical liquid against blood flow and plasticity for not causing damage to a blood vessel wall are required as its functions. A relatively thick-walled tube is used as a core in which stainless steel wires are woven (braided) into a relatively soft resin such as polyester. However, a metal tube is preferred in view of obtaining a pressure-resistant catheter with a small diameter and a thinner wall while a superelastic Ti-Ni alloy having high plasticity is suitable in view of functionality. The present inventors conducted studies for aiming practical use of a catheter including a Ni-rich Ti-Ni-alloy (Ti-51 at% Ni) tube which can readily show superelasticity at a body temperature. However, problems remain to be solved such as ensuring softness for passing through a branched blood vessel, formation of a knife-edged fracture surface upon fracture and the like. Therefore, it is not yet available commercially. (Patent Documents 1 to 2)

A stent is a mesh-like metal pipe to be indwelled inside the body in order to prevent restenosis after expanding a narrowed segment of a blood vessel and the like. A collapsed stent accommodated in a front end part of a catheter is first introduced to a narrowed segment, and then operated to be released from the catheter and expanded to engage against the inner wall of a lumen such as a blood vessel.

A narrowed segment in the coronary artery which may cause myocardial infarction and the like can be expanded according to the vasodilation procedure through inflating a balloon placed in the storage inner wall of a stent. This is called a balloon expandable type, in which stainless steel and a cobalt-chromium alloy are used as a metal therein. There are a large number of prior literatures, including Patent Document 3 in which Palmaz yielded the first practical application in the world in 1988, and subsequent documents in which balloon-expandable types were put into practical use. Meanwhile, a self-expandable stent, which can spontaneously restore the shape immediately after released from a catheter, is used for expanding a narrowed segment in the carotid artery which may trigger cerebral infarction. For a metal therein, used is a superelastic Ti-Ni alloy material having excellent spring properties. (Patent Documents 3 to 8)

Shape memory alloys including a Ti-Ni alloy are well known to show significant shape memory effects associated with reverse transformation of the martensitic transformation. Further, they are also well known to show good superelasticity associated with the stress-induced martensitic transformation caused by deformation after reverse transformation. These functionalities are manifested in connection with temperature cycles of cooling/heating, and the shape recovery temperatures in the case of heating are classified into the starting temperature (the As temperature) and the finish temperature (the Af temperature). The functionality manifestation of superelasticity finishes at a temperature of the Af temperature or above. Among many shape memory alloys, Ti-Ni alloys and Ti-Ni-X alloys (X = V, Cr, Co, Nb, Cu and the like) in particular can show significant superelasticity of this type. The practical uses of Ti-Ni alloys are increasing not only in the medical field related to the present application but also in a wide range of fields such as appliance, automobile, clothes and construction. Moreover, many of the technologies related to Ti-Ni-alloys have matured to the point of the establishment of standard engineering specifications, and are utilized for describing important specifications. As an example, the scope of Ti-Ni alloys is defined as Ni: 53.5 to 57.5 mass% (48.5 to 52.5 mol%) alloys according to JIS H-7101 "Wires, tapes and tubings of Ti-Ni shape memory alloy". (Nonpatent Document 1)

Further, Ni-rich Ti-Ni alloys are well known to generate precipitates of TiNi₃, Ti₃Ni₄ and the like when aging treatment is performed, affecting transformation properties and mechanical properties of an element. For example, the transformation temperature of a Ti-51 at% Ni alloy material subjected to aging treatment is shifted to the side of higher temperature because the concentration of Ni in a matrix is decreased due to the generation of excessive Ni precipitates. The Af temperature of a TiNi single phase is 0°C or less when solution treatment is performed whereas the Af temperature increases to approximately 25°C when aging is performed at 500°C for 2 hours. In this regard, the mechanical properties thereof are improved, showing good superelasticity (excellent in repetition with less significant hysteresis). It is known that precipitation of TiNi₃, Ti₃Ni₄ and the like in a Ti-Ni alloy occurs in a case where the concentration of Ni is 50.5 at% or more, and the aging temperature is approximately 400°C or more (Nonpatent Document 2). However, a case in which the aging temperature is less than approximately 400°C has not been particularly described.

A Ti-Ni alloy used for a catheterization device typically has an Ni-rich composition and is limited to the Ti-51 at% Ni alloy. In view of the necessity of superelasticity well manifested at the room temperature and body temperature, a core preferably includes a material having a shape recovery temperature equal to or lower than the body temperature when solution treatment is performed. The aforementioned alloy, which can maintain the manifestation of superelasticity at the body temperature regardless of heat treatment conditions, is widely used as a base material of the above device, and used in virtually every commercial product currently available for practical use.

A guide wire includes a solid wire material as its core, which can be manufactured in the same way as common steel materials according to a method selected from many alternatives such as severe plastic deformation and straightening. Properties required for a given application can optionally be provided. However, a front end part, which needs to be flexible not causing damage to a blood vessel, relies on taper processing mainly by grinding and etching. However, the strength of the front end part decreases as the cross sectional area decreases. This brings an issue of fracture. For this reason, a front end part pressed into a ribbon-like shape, in which the cross sectional area is not decreased, has been proposed, but it differs from a non-directional circular cross section having directional softness. Softness is preferably assured in a straight cross section.

The cores of catheters and stents are commonly a hollow pipe, and the straightness thereof is essential for manufacturing the devices. Heat straightening is often performed at a temperature near the recrystallization temperature after severe plastic deformation. In that case, it is essentially inevitable that a material which shows superelasticity at the body temperature is limited to the Ti-51 at% Ni alloy. Further, in the case of a catheter, mechanical strength can be controlled by cold working and heat treatment after heat straightening, but disadvantageously, it is susceptible to self-collapsing when U- and V-shape bending deformation upon passing through a curved blood vessel and when pressed and crushed. A breakage surface thereof tends to show a knife-like fracture surface. This trend is particularly significant for the Ti-51 at% Ni alloy.

Furthermore, a stent core widely used for practical use as a superelastic material also includes an Ni-rich Ti-Ni alloy (for example; Ti-51 at% Ni), and is subjected to aging treatment at 400 to 500°C after cold working. This is responsible for the functional manifestation of superelasticity with various characteristics, and satisfies the basic requirements for generating precipitates of Ti₂Ni₃ and the like. However, repetition fatigue remains problematic as compared with a Ti-Ni single phase material subjected to solution treatment although relatively high yield strength is maintained.

### Citation List

### Patent Literature

Patent Document 1: Japanese Patent Application Laid-Open No. H2-144074
Patent Document 2: Japanese Patent Application Laid-Open No. H4-28375
Patent Document 3: US Patent No. 4733665
Patent Document 4: Japanese Patent Application Laid-Open No. H06-054913
Patent Document 5: Japanese Patent Application Laid-Open No. H08-000738
Patent Document 6: Japanese Patent Application Laid-Open No. H11-99207
Patent Documents 7: Japanese Patent Application Laid-Open No. 2005-245848
Patent Documents 8: Japanese Patent Application Laid-Open No. 2006-325613

### Nonpatent Documents

Nonpatent Document 1: JIS H-7101
Nonpatent document 2: Kazuhiro Otsuka, "Martensitic transformation and shape memory effects of alloys" Uchida Rokakuho Publishing Co., Ltd., (2012), p194

### Summary

### Technical Problem

An objective of the present invention is to provide a medical alloy usable for cores of catheters and guide wires in which both strength and plasticity at the body temperature are maintained. That is, provided is a breakage-safe core allowing easy passage through a curved blood vessel and a narrowed segment and having bending restorability after passage, which is less susceptible for breakage even bent in U- and V-shape manners, and tends not to form a knife-like fracture surface. Moreover, another objective is to provide a stent core for a stent which is excellent in breakage-safety after indwelled inside the body, sustained high expandability for indwelling and sheath accomodability and operatability.

### Solution to Problem

The present investors found that precipitates, which otherwise favorably affect mechanical properties of an alloy, were actually responsible for breakage/formation of a knife-edged fracture surface in a medical device such as a catheterization device. The present inventors further found that the above problems could be solved by providing a core element largely including a Ti-Ni single phase to reduce the influences of precipitates. Then the present invention has been completed.

That is, according to the present invention, superelasticity can be manifested at the body temperature under conditions where precipitates are not formed even in a case where aging treatment is performed. One embodiment of the present invention is a Ti-Ni alloy subjected to thermomechanical treatment in which precipitates such as Ti₃Ni₄ are not easily formed, or a Ti-Ni alloy having a composition of Ti-50.0 to 50.5 at% Ni. Further, the Ti-Ni alloy according to the present invention is a material having an Af temperature of more than 37°C due to solution treatment of a TiNi single phase.

### Advantageous Effects of Invention

The alloy according to the present invention can provide a medical Ti-Ni shape memory alloy with a core superelastic at the body temperature, and a medical device such as a catheter made use of the above alloy. The above alloy and medical device have the following advantages as compared with the conventional products: formation of a knife-edged fracture surface can be avoided when bending and braking; a strength at initial deformation can optionally be provided; and the like.

Moreover, according to the present invention, a catheter, a guide wire in which a part of the device shows superelasticity at the body temperature, and another part shows shape memory at the body temperature can be obtained. Further, the front end part of the device or a portion thereof may have a relatively low stiffness (less than 300 MPa) and can easily be reformed. In addition, the base or a portion thereof may have a relatively high stiffness (500 MPa or more).

### Brief Description of Drawings

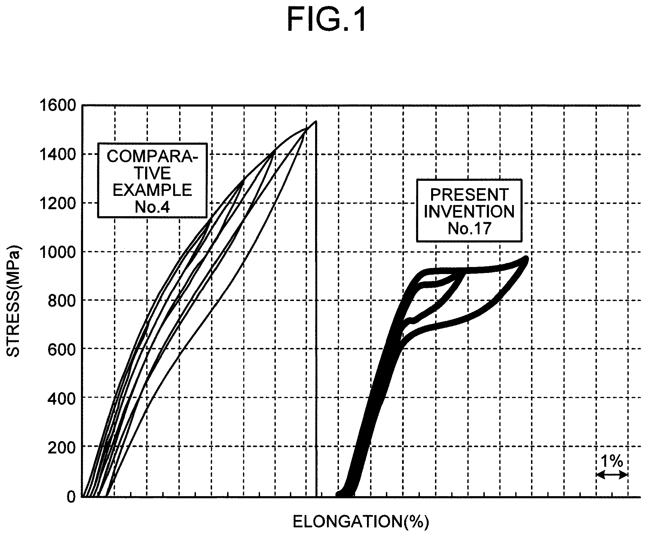

Fig. 1 illustrates examples of tensile tests for Ti-Ni alloys. Illustrated are results from tensile tests in Comparative Example No.4 and Example 17 of the present invention in Table 1. The present invention shows superelasticity with a yield plateau whereas Comparative Example does not show clear yield.

### Description of Embodiments

According to the present invention, a Ti-Ni alloy largely including a TiNi single phase in which precipitation of compositions other than that is restrained is used as a core that is superelastic at the body temperature. As shown in Nonpatent Document 2, Ti₃Ni₄ precipitates in a Ti-Ni alloy in a case where the concentration of Ni is more than 50.5 at%, and the aging temperature is approximately 400°C or more. Therefore, the key to the present invention is to form a core outside the above conditions.

Therefore, in order to obtain a Ti-Ni alloy largely including a TiNi single phase, it is specifically necessary that various processing treatments are performed on a composition of a Ti-Ni alloy in a range of Ti-50.0 to 50.5 at% Ni, or aging treatment after solution treatment of a Ti-Ni alloy is performed at less than 400°C.

Further, the present inventors found that the mechanical properties of a Ti-Ni alloy having a composition of Ti-50.0 to 50.5 at% Ni were extremely dependent on a heat treatment temperature, and the strength at initial deformation (for example, at 2% stretching) necessary for a catheterization device could optionally be provided. Further, the present inventors found that a core which was excellent in shape straightness, high radial force and sheath accomodability and operatability could be obtained by subjecting a TiNi single phase element to processing strain and relaxation treatment sufficient to achieve superelasticity with a yield plateau at the body temperature regardless of Ti-Ni alloy compositions.

In the present invention, solution treatment refers to a heat treatment in which an alloy is heated to a specific temperature, and quenched from a state in which alloy elements are in solid solution, and then a high temperature composition is directly brought to ordinary temperature. In solution treatment for transforming a Ti-Ni alloy into a TiNi single phase, a temperature of around 800°C is sufficient for the aforementioned heating temperature.

In the present invention, aging treatment refers to a heat treatment in which an alloy is again heated after solution treatment. Depending on heating conditions and the composition of an alloy, precipitates may be formed in the alloy, allowing the mechanical properties thereof to be altered. In the present invention, aging treatment is preferably performed under conditions of at less than 400°C and for relatively a short time (in the present Example, 30 minutes or less), but the conditions are not limited to these in a case where the composition of a Ti-Ni alloy is Ti-50.0 to 50.5 at% Ni.

In the present invention, restraining precipitation of a composition into a TiNi phase means reducing generation of precipitates of TiNi₃, Ti₃Ni₄ and the like in the core of an alloy, and also means obtaining a state close to a TiNi single phase. Treatments required to achieve these include, for example, making Ti-Ni alloy with a composition within a range of Ti-50.0 to 50.5 at% Ni and performing aging treatment on a under temperature conditions of less than 400°C after solution treatment; and the like.

In the present invention, the body temperature refers to body temperatures of homeothermal animals such as birds and mammals, and in particular preferably the body temperature of humans. The range of the temperature can be any within a range of body temperatures which these animals can usually have. For example, it is preferably within a range of from 35°C to 42°C, and more preferably the temperature is 37°C.

### Example

### [Evaluation of wire]

Table 1 shows transformation profiles and trial manufacture conditions of each wire of Ti-51 at% Ni, Ti-50.5 at% Ni and Ti-50 at% Ni, and the results therefrom.

Each transformation temperature shown in Table 1 results from DSC (differential scanning calorimetry) measurements after performing heat treatment at 800°C where a TiNi single phase is obtained. The Af temperature of the Ti-51 at% alloy from Comparative Examples was found to be-15°C, which is below the body temperature while those of Ti-50.5 at% Ni and Ti-50 at% Ni alloys from Examples of the present invention were found to be 48°C and 105°C, respectively, which are above the body temperature.

Since straightness is necessary for use in devices, trial wires were subjected to tensile treatment at 200°C to 600°C.

Comparative Examples 1, 2, 3 and 4 all show good superelasticity at the body temperature, but a straight wire for use in devices is not obtained unless the treatment temperature is near recrystallization, and variations in tensile strength (ε = 2%) were less significant under the treatment conditions. This means that stable superelasticity can easily be obtained regardless of manufacturing methods, but options for properties are limited, and the strength is dictated by selection of a wire cross section.

The table shows that bending fracture is significant for Comparative Examples 2, 3 and 4, showing that they are not suitable for medical use. Further, not shown in the table, Comparative Example 1 has inferior durability due to the absence of transition as compared with other Comparative Examples and Examples of the present invention. Therefore, it is not suitable for medical use although bending fracture (formation of a knife-edged fracture surface) is less significant.

In contrast, Examples 5 to 16 of the present invention show superior processability, and thus suitable straight wires can be obtained by performing a treatment such as straightening treatment at 200°C after cold working. Further, in terms of properties, a variety of superelasticity at 37°C profiles, selections of shape memory and tensile strengths can be selected depending on conditions of the heat treatment. Moreover, these trends are significant for the equiatomic composition Ti-50 at% Ni alloy. Furthermore, fracture/formation of a knife-edged fracture surface when bending are not observed or less significant for all test pieces, showing remarkable difference from Comparative Examples. Further, No. 17 and 18 according to the present invention, which are products obtained by subjecting the Ti-51 at% Ni alloy from Comparative Example to SW (swaging) processing where straightness can be obtained relatively easily, satisfy requirements of the present invention such as finishing shapes and strength.

**Table 1 Evaluation results of trial Ti-Ni-allov wires**

| | Trial wire | | | | Property | | | | |
|---|---|---|---|---|---|---|---|---|---|
| | No | Alloy | Processing rate | Tension treatment (Aging treatment) | 37°C profile | Yield plateau | Straightening | Bending fracture | Tensile strength MPa (ε 2%) |
| Comparative Examples | 1 | Ti-51 at% Ni Af: -15°C (Solution treatment) | 30% Die processing | 600°C/5 min | Super elastic | Good | Good | Less significant** | 500 |
| | 2 | | | 400°C/10 min | Super elastic | Good | Good | Significant | 500 |
| | 3 | | | 300°C/30 min | Super elastic | Bad | Bad | Significant | 600 |
| | 4 | | | 200°C/30 min | Super elastic | Bad | Bad | Significant | 800 |
| | 5 | Ti-50.5 at% Ni Af: 48°C (Solution treatment) | 30% Die processing | 600°C/5 min | Shape memory | - | Good | Less significant | 300 |
| | 6 | | | 400°C/10 min | Super elastic | Good | Good | Less significant | 320 |
| | 7 | | | 300°C/30 min | Super elastic | Good | Good | Less significant | 320 |
| | 8 | | | 200°C/30 min | Super elastic | Bad | Good | Less significant | 500 |
| Examples of Present Invention | 9 | | 40% Die processing | 500°C/5 min | Shape memory | - | Good | Less significant | 150 |
| | 10 | | | 400°C/10 min | Shape memory | - | Good | Less significant | 200 |
| | 11 | Ti-50 at% Ni Af: 105°C (Solution treatment) | | 300°C/30 min | Super elastic | Good | Good | Less significant | 350 |
| | 12 | | | 200°C/30 min | Super elastic | Bad | Δ* | Less significant | 600 |
| | 13 | | 60% Die processing | 600°C/5 min | Shape memory | - | Good | Less significant | 150 |
| | 14 | | | 400°C/10 min | Shape memory | - | Good | Less significant | 300 |
| | 15 | | | 300°C/30 min | Super elastic | Good | Good | Less significant | 600 |
| | 16 | | | 200°C/30 min | Super elastic | Bad | Δ* | Less significant | 1000 |
| Present Invention | 17 | Ti-51 at% Ni Af:-15°C (Solution treatment) | 40% SW processing | 350°C/1 min | Super elastic | Good | Good | Less significant | 900 |
| | 18 | | | 300°C/30 min | Super elastic | Good | Good | Less significant | 900 |

| | | | | | | | | | |
|---|---|---|---|---|---|---|---|---|---|
| Δ* Depends on the finishing shape. ** Durability was inferior due to the absence of transition. | | | | | | | | | |

### [Guide wire and catheter]

Basic functional tests were performed on guide wires and catheters including alloys shown in Table 1. The results are shown in Table 2.

Finishing treatment for the materials in the table is cold working of approximately 30%. End part processing of a trial core means a treatment for reducing a diameter in order to obtain the plasticity of the front end part of a guide wire. Moreover, in the property evaluation, breakage represents a fracture profile upon V-bend press-crushing, and end part reshaping represents a reforming profile without superelasticity.

**Table 2 Evaluation of trial cores for guide wire and catheter**

| | No | Alloy | Trial Core | | | | Property | | | |
|---|---|---|---|---|---|---|---|---|---|---|
| | | | Material | Base part treatment | End part treatment | End part processing | Base Part | | EndPart | |
| | | | | | | | Function | Breakage | Plasticity | Reshaping |
| Comparative Example | 17 | Ti-51 at% Ni | φ 0.5 mm Wire | 400°C | 400°C | φ 0.1 | Super elastic | Significant | Bad | X |
| | 18 | Ti-51 at% Ni | φ0.5 mm Wire | 400°C | 600°C | - | Super elastic | Significant | Bad | Bad |
| | 19 | Ti-51 at% Ni | φ1.0 mm Tube | 400°C | 400°C | - | Super elastic | Significant | Bad | Bad |
| Present Invention | 20 | Ti-50.5 at% Ni | φ0.5 mm Wire | 300°C | 600°C | 0.1 | Super elastic | Less significant | Good | Δ* |
| | 21 | Ti-50 at% Ni | φ0.5 mm Wire | 300°C | 600°C | φ 0.2 | Super elastic | Less significant | Good | Good |
| | 22 | Ti-50 at% Ni | φ1.0 mm Tube | 300°C | 600°C | - | Super elastic | Less significant | Good | Good |

| | | | | | | | | | | |
|---|---|---|---|---|---|---|---|---|---|---|
| Δ* Characteristics of spring somewhat remain. | | | | | | | | | | |

### [Stent]

Basic functional tests were performed on stents including alloys shown in Table 1.

The results are shown in Table 3. Finishing treatment for the materials in the table is cold working of approximately 30%. A trial core was obtained by performing an expanding/shape-fixing treatment in which a raw tube with a diameter of 2 mm was processed into a body-indwelled diameter of 10 mm. The term "sequential expansion" in the property items means a stepwise treatment from φ 2 to φ 10 considering the risk of fracture and completed after about 5 steps for Comparative Examples while completed after about 2 to 3 steps for Examples of the present invention. Further, the presence or absence of shape memory properties at the end part intended whether stimulation of the inner wall of a blood vessel by the end part of an indwelled stent, due to partial superelastic loss, is reduced or whether delivery properties are enhanced.

**Table 3 Evaluation results of stent**

| | No. | Alloy | Trial Core | | | Property | | |
|---|---|---|---|---|---|---|---|---|
| | | | Material | φ 10 expanding treatment | | Sequential expansion φ2 →φ10 | Property | |
| | | | | Base part | End part | | Base part | End part |
| Comparative Example | 17 | Ti-51 at% Ni | φ2.0 mm Slotted tube | 400°C | 400°C | 5 | Super elastic | Super elastic |
| | 18 | Ti-51 at% Ni | φ2.0 mm Slotted tube | 400°C | 600°C | 5 | Super elastic | Super elastic |
| | 19 | Ti-50.5 at% Ni | φ2.0 mm Slotted tube | 400°C | 400°C | 3 | Super elastic | Super elastic |
| Present Invention | 20 | Ti-50.5 at% Ni | φ2.0 mm Slotted tube | 300°C | 600°C | 3 | Super elastic | Shape memory |
| | 21 | Ti-50 at% Ni | φ2.0 mm Slotted tube | 300°C | 400°C | 2 | Super elastic | Shape memory |
| | 22 | Ti-50 at% Ni | φ 2.0 mm Slotted tube | 300°C | 600°C | 2 | Super elastic | Shape memory |

### Industrial Applicability

According to the present invention, a shape memory alloy for medical use can be obtained in which both strength and plasticity at the body temperature are maintained. Further, the present invention can provide a catheter, guide wire and the like including the above alloy, characterized in that, the formation of a knife-edged fracture surface can be avoided when bending and breaking, and a strength at initial deformation can optionally be provided.

## Claims

1. A medical Ti-Ni shape memory alloy, wherein precipitation of a composition other than TiNi into a TiNi phase is restrained.

2. A medical Ti-Ni shape memory alloy, wherein precipitation of a composition other than TiNi into a TiNi phase after aging treatment is restrained.

3. The medical Ti-Ni shape memory alloy according to claim 1 or 2, wherein the aging treatment is performed under temperature conditions of below 400°C after solution treatment.

4. The medical Ti-Ni shape memory alloy according to any one of claims 1 to 3, wherein the medical Ti-Ni shape memory alloy shows a shape-recovery finish temperature of higher than 37°C by performing the solution treatment.

5. The medical Ti-Ni shape memory alloy according to any one of claims 1 to 4, wherein the medical Ti-Ni shape memory alloy is processed by performing structure control so that the medical Ti-Ni shape memory alloy has superelasticity at a body temperature.

6. The medical Ti-Ni shape memory alloy according to any one of claims 1 to 5, wherein a composition of Ti and Ni is Ti-50.0 to 50.5 at% Ni.

7. The medical Ti-Ni shape memory alloy according to any one of claims 1 to 6, wherein the medical Ti-Ni shape memory alloy has superelasticity with a yield plateau at the body temperature.

8. The medical Ti-Ni shape memory alloy according to any one of claims 1 to 7, wherein the medical Ti-Ni shape memory alloy is processed by performing different heat treatments on different parts so that the medical Ti-Ni shape memory alloy includes a part having superelasticity at the body temperature and a part having shape memory at the body temperature.

9. A medical device for endovascular treatment, manufactured by using the medical Ti-Ni shape memory alloy according to any one of claims 1 to 8.

10. A guide wire, catheter, or stent, manufactured by using the medical Ti-Ni shape memory alloy according to any one of claims 1 to 8.
